# EUROPEAN PATENT APPLICATION

(11) **EP 4 011 353 A1**
(43) Date of publication of application: **15.06.2022**
(21) Application number: 20212756.9
(22) Date of filing: 09.12.2020
(51) Int. Cl.: A61K 8/34, A61K 8/44, A61Q 5/00, A61Q 17/04, A61Q 19/00, A61K 8/365, A61K 8/67

(54) **NEW COSMETICS SOLVENTS COMPRISING ASCORBIC ACID**

(71) Applicant: Beiersdorf AG, 20253 Hamburg (DE)
(72) Inventor: Piacentini, Adalberto, 22529 Hamburg (DE); Köhler, Manuela, 22527 Hamburg (DE); Kallmayer, Volker, 22393 Hamburg (DE); Bernau, Mareike, 22087 Hamburg (DE); Ponnudurai, Arulselvan, 22523 Hamburg (DE)
(74) Representative: Beiersdorf AG

(57) **Abstract**

The invention is an eutectic solvent formed from the mixture of ascorbic acid (vitamin C) in combination with Betaine and a third component selected from the group comprising Water, Ethanol, Glycerol, Diols and/or Triols with 6 or less than 6 C-atoms, especially 1,3 - Propanediol, Butylene Glycol and Hexanediol. The new inventive eutectic solvents allows an improved incorporation of active ingredients into cosmetic compositions.

## Description

The invention is an eutectic solvent formed from the mixture of ascorbic acid (vitamin C) in combination with Betaine and a third component selected from the group comprising Water, Ethanol, Glycerol, Diols and/or Triols with 6 or less than 6 C-atoms, especially 1,3 - Propanediol, Butylene Glycol and Hexanediol. The new inventive eutectic solvents allow an improved incorporation of active ingredients into cosmetic compositions.

Natural deep eutectic solvents (NADES) are bio-based ionic liquids and deep eutectic solvents which are composed of two or more compounds that are generally plant based primary metabolites, i.e. organic acids, sugars, alcohols, amines and amino acids. Ionic liquids are salts, liquid at room temperature, characterized by ionic bonds which have at least one large organic ion and a cation with a low degree of symmetry. The positive and negatively charged ions in the liquid are thus kept far apart so that they reduce the attractive forces between them and hinder crystallization, lowering the melting point and resulting in a completely ionic liquid at room temperature. Deep eutectic solvents (DES) are mixtures of solid compounds that form liquids due to a large depression of the melting point; the driving force in the case of DES is the extensive hydrogen bonding between components forming a non-aqueous liquid at room temperature. Work done by Choi, Spronsen *et al.* showed that water can be present as part of the solvent, being strongly retained in the liquid and which cannot be evaporated.

FR 3036618 describes NADES, methods in which these NADES are involved, plant extracts obtained by using the NADES and the use of these plant extracts for the preparation of cosmetic compositions.

The NADES are liquid at room temperature and characterized in that they consist of a first and a second compound, each selected from the group comprising the family of polyols, the family of sugars or the family of amino acids and that the first compound and the second compound are selected from different families, the NADES may advantageously further comprise an aqueous phase.

FR 3017292 describes a cosmetic formulation based on vegetable or natural origin, a cosmetic composition containing the formulation, and a process for the preparation of such a cosmetic composition. It also relates to the use of a eutectic solvent as the basis of a cosmetic formulation and to a device comprising a cosmetic composition. The cosmetic formulation base comprises a eutectic solvent consisting of natural or plant molecules. The mixture of a eutectic solvent and of an active ingredient until a homogeneous dispersion is obtained and the resulting homogeneous dispersion is cooled until a balsam texture is obtained.

FR 3046352 describes the cosmetic use of a eutectic solvent consisting of molecules of vegetable or natural origin, as an agent that enhances the barrier function of the epidermis to improve the radiance of the skin, and / or smooth the skin surface and / or smooth the skin and / or improve the complexion of the skin.

The eutectic solvent comprises at least one active ingredient selected from mangiferin, mangustin, rutin, apigenin, esculin, mannitol, Baaleinin lupeol acetate, coumarins, polyols, triterpenes, saponins, carotenoids, polyphenols or a mixture thereof or plant extracts rich in these molecules, such as an ash manna extract (fraxinus ornus) rich in mannitol, an extract of chicle gum (Manilkara zapota) rich in lupeol acetate, an extract of scutellar (Scutellaria baïcalensis), rich in bayalein or an extract of green coffee beans, which are rich in chlorogenic acids.

The following problems need to be improved or resolved.

Active ingredients are normally incorporated into cosmetic products as a solution in water. However, after application, water evaporates quickly and the actives remaining in solution precipitate. Once the actives are precipitated, they are no longer bioavailable and display no more activity.

Vitamin C (ascorbic acid) is one of the common active ingredients and a compound with proven biological activity for cosmetic application, however it decomposes in presence of water and air, typically in the span of a few days to a few weeks. This behaviour limits its incorporation to cosmetically acceptable and long-term stable formulas.

Recently there has been extensive studies about the possibilities of achieving better skin properties by ensuring the microbiome of the skin is well balanced. One strategy is to deliver living bacteria which display a positive effect on the microbiome. However, it is a challenge to find a suitable medium where the desired bacteria strain remains alive and in the desired concentration, that means no growth or death.

Another problem seems to be that some compounds needed to ensure the functionality of a cosmetic formulation, which can penetrate the skin barrier and leading to undesired biological effects. Their activity is only desired on the external surface of the skin - however there should be little possibilities of decreasing their penetration without modifying their chemical structure.

In addition, long-last moisturization of the skin has always being a cosmetic challenge, specially taking sensory of the final formula into consideration. Water-binding substances such as glycerine, propanediol and urea are commonly used, but their alternatives are still relatively scarce. New substances capable of enhancing the perceived moisturization of the skin are needed in order to expand the formulation possibilities.

All these challenges and problems are solved by the inventive eutectic solvent or by a composition, especially cosmetic composition, comprising such an eutectic solvent which is formed from the mixture of ascorbic acid (vitamin C) in combination with Betaine and a third component selected from the group comprising water, ethanol, glycerol, diols and/or triols with 6 or less than 6 C-atoms, esp. 1,3 - Propanediol, Butylene Glycol and Hexanediol.

Surprisingly, in addition to solubilizing and protecting vitamin C in different NADES, another new inventive strategy was created, wherein ascorbic acid is one of the molecules forming the NADES itself.

With this approach, the following NADES were created by combining betaine, vitamin C and a third component:
The preferred molar ratios were selected between ascorbic acid (a) : Betaine (b) : third component (c) from a to b to c wherein a, b and c were selected in the range from 1 to 10, preferred in the range from 1 to 5.

Preferred inventive eutectic solvents are
- Vitamin C + Betaine + Water,
- Vitamin C + Betaine + Glycerol and
- Vitamin C + Betaine +1,3-Propanediol

The preferred molar ratios and mass compositions of these inventive eutectic solvents are the following:

| 3^{rd} component | Molar ratio Vitamin C:Betaine:3^{rd} component | Composition (in wt. %) |
|---|---|---|
| Water | 1:1.2:2.6 | 49% Vitamin C |
| | | 38% Betaine |
| | | 13% Water |
| Glycerol | 1:2:4.6 | 21% Vitamin C |
| | | 29% Betaine |
| | | 50% Glycerol |
| Glycerol | 1:2:10 | 13% Vitamin C |
| | | 17% Betaine |
| | | 70% Glycerol |
| 1,3-Propanediol | 1:2:5.5 | 21% Vitamin C |
| | | 29% Betaine |
| | | 50% Propanediol |

The preferred compositions, the one containing the least amount of water and still being stable for months at room temperature, is the following:
49% wt. vitamin C + 38% betaine + 13% water

Also, with this strategy, a completely new composition is developed with the following composition:
21% wt. vitamin C + 29% betaine + 50% glycerol

This is equivalent to a concentration of 0.42g of vitamin C per g of glycerin. The saturation concentration was measured below 0.06 g of vitamin C per g of glycerin.

This allows the incorporation of higher contents of vitamin C (preferably up to 21%) under its ascorbic acid form in hydrophilic formulations, instead of using ascorbic acid derivatives or recurring to the usage of its powder form.

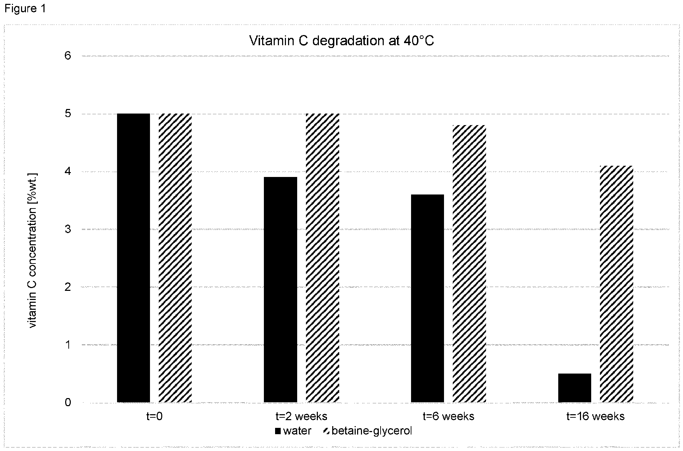

As an example of decreased degradation of vitamin C dissolved in inventive eutectic solvents compared to water, the stability of 5 % vitamin C solutions was followed at 40 degrees for 16 weeks, leading to a surprisingly enhanced protection of vitamin C as part of the inventive eutectic solvents, as shown in figure 1.

Figure 1 shows the degradation of vitamin C at 40°C in water compared to the composition according to the invention (5% vitamin C, 40% betaine, 55% glycerol).

In a further test, the stability of vitamin C in water and in the preparation according to the invention was examined.

Figure 2 shows photos representing the color stability of Vitamin C NADES technology (21% vitamin C, 29% betaine, 50% glycerin) compared with 21% vitamin C in water.

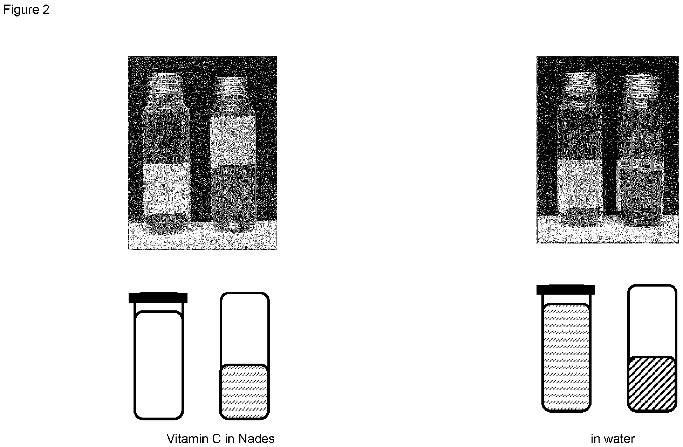

Figure 2 shows closed (left) and open (right) glass flasks after 3 months at room temperature, including 21% vitamin C in water and in NADES.

The experiment shows that vitamin C is unstable in water both in opened glass bottles and in closed bottles. Whereas vitamin C remains stable in closed bottles in betaine and glycerol.

The inventive eutectic solvents or a composition comprising the eutectic solvents shows
- a new way to increase the availability of actives by solving them in new hydrophilic solvents which do not evaporate;
- a surprising new way of delivering ascorbic acid in a liquid form with reduced degradation compared to water solution;
- a remarkable boost and long-lasting moisturization when compared to usual cosmetic strategies;
- a new hydrophilic bacteriostatic solvent, in which gram positive and gram-negative bacteria can remain dormant and be reactivate by simple dilution in water;
- a solvent that hinders the penetration of cosmetic compounds which activity is expected on the surface of the skin and dermal absorption is not desired

Surprisingly, these new class of solvents was developed by combining three substances selected from the group of ascorbic acid, betaine and a third component, preferred selected form the group consisting of water, ethanol, glycerol, diols and/or triols with 6 or less than 6 C-atoms, esp. 1,3 - Propanediol, Butylene Glycol and Hexanediol.

Preferably these new class of solvents was developed by combining three substances in precise molar ratio leading to the formation of a liquid phase displaying a melting point lower than the individual compounds. In most of the cases, the new liquids do not crystallize as pure substances does, but rather become amorphous solids upon cooling. Therefor no melting point can be determined experimentally, only a glass transition temperature. But these new solvents display a negligible vapor pressure, therefore they do not evaporate at room temperature and pressure.

The new inventive eutectic solvents allow an improved incorporation of active ingredients in addition to ascorbic acid into cosmetic compositions. The active ingredient is stabilized and more readily available.

Therefore, the amount of active ingredients can be reduced in the composition without of a decrease of activity.

And, the other way, it is possible to add more quantity of actives and therefore have more bioavailability.

As preferred the following active ingredients could be added to the inventive composition:
Glycyrrhiza Inflata Root Extrakt, Silver Citrate, 4-[(Cyclopentylhydroxyphenylacetyl)oxy]-1,1-dimethyl-piperidiniumbromid (GPIP), Isobutylamido Thiazolyl Resorcinol (Thiamidol), Sodium Ascorbyl Phosphate, Folic acid, Tocopherol, Lipoic acid, Kalium-Methoxysalicylate, Vitamin B6 HCI, Pyrus Malus Stem Extract, Glyceryl Glucoside, short and / or long chain hyaluronic acid, 4-Butyl-Resorcin, Octadecene Dioic acid, Caffeine, Carnitine, Creatine, Cystine, I-tyrosine, Alpha-Glucosylrutin, Taurine, Glycyrrhizic acid, N-Acetylhydroxyprolin, Niacinamide, Magnesium-Ascorbylphosphat, Ellagic acid, licorice root extract, sea salt, Isoserinol, Ascorbylglucosid, Undecenoylphenylalanin, Kojic acid, arbutin, Zingerone, Dihydromyricetin, 4-Hexyl-Resorcin, Phenylethyl Resorcinol, Ubiquinone, esp. Q10, Cyanomethylphenyl Menthan Carboxamide, Menthoxypropanediol, Menthan Carboxamide Ethylpyridin, Hydroxyethylurea, Climbazole, Epsilon-Poly-L-Lysine, Laureth-9, Piroctone Olamine, Selenium sulfide, zinc pyrithione as well as deodorant- and/or antiperspirant active ingredients and as well as plant- and/or fruit extracts.

The solubility of actives is higher in inventive eutectic solvents than in water and contrarily to oils, these solvents are hydrophilic and have the same (or even superior) capacity to dissolve actives, like folic acid, Thiamidol or even other molecules like tapioca starch.

Water in contact with these liquids is often in a remarkably bound state due to extensive hydrogen bonding interactions. Some of these liquids integrated into cosmetic formulas lead to an improved moisturization of the skin due to extensive water binding.

Surprisingly, the following advantages based on the eutectic solvents according to the invention, which are explained in more detail below.

The combination of the solid ascorbic acid with the other inventive eutectic solvent components surprisingly yields a liquid due to the decrease in the melting point. This liquid, stable at room temperature, can be incorporated into cosmetic formulas. Furthermore, it was shown that the addition of water or ethanol up to 15%, preferably up to 10%, can be used as a strategy to decrease the viscosity of the liquid without increased degradation of the ascorbic acid. This lead to decreased vitamin C degradation compared to water solutions used nowadays.

The combination of the inventive compounds leads to a mixture displaying a melting point significantly inferior to the individual compounds and liquid at room temperature. The mechanism behind is related to a strong hydrogen-bonding capacity between the molecules. This strong molecular interaction can happen also with molecules solubilized in the mentioned liquid, leading to a decrease in the liberation and dermal-absorption of the compound during pig-skin penetration test.

The eutectic solvents according to the invention shows a reservoir effect. When dissolved in the aqueous phase, actives tend to lose their bioactivity whenever water evaporates, due to their crystallization. By dissolving the actives of interest in the new eutectic solvents, an increase in the availability is observed, leading to a better dermal absorption.

Betaine is a quaternary ammonium compound with three methyl groups and structure

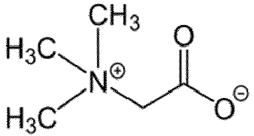

Melting point of betaine is 301 °C.

Ethanol is an organic chemical compound with the structure 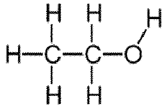 and melting point of -114 °C.

Glycerol (Glycerin, Propane -1,2,3 -triol) is a common cosmetic ingredient.

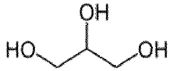

Melting point of glycerol is 18°C.

1,3- Propanediol belongs to the group of dihydric alcohols, diols.

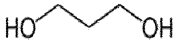

Melting point of 1,3- propanediol is -26°C.

Butylene Glycol, 1,3-Butanediol, is a common cosmetic ingredient.

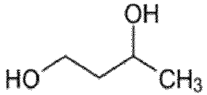

Melting point of Butylene glycol is -50°C.

Hexanediol (1,2-Hexanediol) is a moisturizing, common cosmetic ingredient.

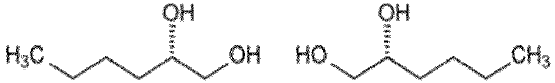

The inventive solvents show a surprising and improved possibility to incorporate active ingredients into cosmetic compositions.

The inventive composition comprising one or more of an eutectic solvents is preferably a part of a cosmetic composition, especially part of a cosmetic composition based on a water in oil emulsion.

Preferably, the cosmetic composition is based on a serum, especially the serum comprises only the three ingredients.

The inventive solvent and composition could be used for improving the incorporation of active ingredients into cosmetic compositions and also for improving the stability of active ingredients in cosmetic compositions and the availability of active ingredients from cosmetic compositions.

The inventive composition could be used for an improved moisturization of the skin.

## Claims

1. An eutectic solvent formed from the mixture of ascorbic acid (vitamin C) in combination with Betaine and a third component selected from the group comprising Water, Ethanol, Glycerol, Diols and/or Triols with 6 or less than 6 C-atoms, especially 1,3 - Propanediol, Butylene Glycol and Hexanediol.

2. Solvent according to claim 1 wherein molar ratio between ascorbic acid (a) : Betaine (b) : third component (c) is chosen from a to b to c, wherein a, b and c were selected in the range from 1 to 10, preferred in the range from 1 to 5.

3. Solvent according to claim 1 or 2 **characterized in that** the eutectic solvent is selected from Vitamin C, Betaine and Water; Vitamin C, Betaine and Glycerol and/or Vitamin C, Betaine and 1,3-Propanediol.

4. A composition comprising one or more of an eutectic solvent according to one of the claims 1 to 3.

5. Composition according to claim 4 as a cosmetic composition.

6. Composition according to claim 5 wherein the cosmetic composition is based on a water in oil emulsion.

7. Composition according to claim 5 wherein the cosmetic composition is based on a serum.

8. Composition according to claim 4, 5, 6 or 7 **characterized in that** the eutectic solvent comprises water in an amount up to 15 percent by weight, based on the total mass of the composition.

9. Composition according to one of the claims 3 to 8 comprising one or more active ingredients, especially cosmetic active ingredients.

10. Composition according to claim 9 wherein the active ingredients were chosen from Glycyrrhiza Inflata Root Extrakt, Silver Citrate, 4-[(Cyclopentylhydroxyphenylacetyl)oxy]-1,1-dimethyl-piperidiniumbromid (GPIP), Isobutylamido Thiazolyl Resorcinol (Thiamidol), Sodium Ascorbyl Phosphate, Folic acid, Tocopherol, Lipoic acid, Kalium-Methoxysalicylate, Vitamin B6 HCI, Pyrus Malus Stem Extract, Glyceryl Glucoside, short and / or long chain hyaluronic acid, 4-Butyl-Resorcin, Octadecene Dioic acid, Caffeine, Carnitine, Creatine, Cystine, I-tyrosine, Alpha-Glucosylrutin, Taurine, Glycyrrhizic acid, N-Acetylhydroxyprolin, Niacinamide, Magnesium-Ascorbylphosphat, Ellagic acid, licorice root extract, sea salt, Isoserinol, Ascorbylglucosid, Undecenoylphenylalanin, Kojic acid, arbutin, Zingerone, Dihydromyricetin, 4-Hexyl-Resorcin, Phenylethyl Resorcinol, Ubiquinone, esp. Q10, Cyanomethylphenyl Menthan Carboxamide, Menthoxypropanediol, Menthan Carboxamide Ethylpyridin, Hydroxyethylurea, Climbazole, Epsilon-Poly-L-Lysine, Laureth-9, Piroctone Olamine, Selenium sulfide, zinc pyrithione, deodorant and/or antiperspirant active ingredients and plant- and/or fruit extracts.

11. Use of a solvent according to one of the claims 1 to 3 for improving the incorporation of active ingredients into cosmetic compositions.

12. Use of a solvent according to one of the claims 1 to 3 for improving the stability of active ingredients in cosmetic compositions.

13. Use of a composition according to one of the claims 4 to 10 for improving the availability of active ingredients from cosmetic compositions.

14. Use of a composition according to one of the claims 4 to 10 for an improved moisturization of the skin.
